Europäisches Patentamt

European Patent Office

Office européen des brevets

(11) Publication number: 0 293 152
A2

# EUROPEAN PATENT APPLICATION

(21) Application number: 88304645.0

(22) Date of filing: 23.05.88

(51) Int. Cl.⁴: A01N 57/34 , A01N 57/20 , C02F 1/50

(30) Priority: 26.05.87 GB 8712372

(43) Date of publication of application:
30.11.88 Bulletin 88/48

(84) Designated Contracting States:
AT BE CH DE ES FR GB GR IT LI LU NL SE

(71) Applicant: Albright & Wilson Limited
210-222 Hagley Road West
Oldbury Warley West Midlands B68 0NN(GB)

(72) Inventor: Smith, Brian
10 Foxhills Park Netherton
Dudley West Midlands DY2 0JO(GB)
Inventor: Talbot, Robert Eric
25 Sunfield Road
Cannock Staffordshire WS11 1NJ(GB)

(74) Representative: Savidge, Roger Gordon
Madgwick et al
c/o Albright & Wilson Limited 1
Knightsbridge Green
London SW1X 7QD(GB)

(54) Method of treating ecologically sensitive water systems.

(57) Ecologically sensitive water systems such as fish ponds, lakes, streams, or aquaria are treated to remove algae with an algicidal, non-ichthyotoxic amount of a water soluble phosphorus compound of the formula

$$[HOR\ R^1_nP]_yX_x$$

wherein R is an alkylene group having 1 to 4 carbon atoms; $R^1$ is HOR, where R has the same significance as above, or an alkyl or alkenyl group having 1 to 4 carbon atoms; n is 2 or 3; x is 1 or 0 such that (n + x) is 2 or 4; y is 1 when x is zero or is equal to the valency of X when x is 1; and X is an anion; or a water soluble condensate of such a phosphorus compound.

# METHOD OF TREATING ECOLOGICALLY SENSITIVE WATER SYSTEMS

The present invention relates to a method of treatment of ponds, lakes, drainage and irrigation ditches, canals, rivers, water-courses, aquaria, fish farms, watercress beds, reservoirs effluents, aqueous discharges from sewage farms or industrial installations and water systems containing, or liable to contain, algae and live fish or other higher organisms to inhibit algal growth.

The various water systems described above, which will be collectively referred to hereinafter as "ecologically sensitive water systems", frequently suffer excessive growth of algae, with detrimental consequences for their appearance, navigability, capacity to support other life forms and/or commercial or amenity value.

The clearing of algal growth is usually a costly and labour-intensive operation and, in some cases, impossible in practice. Chemicals cannot normally be employed to kill the algae because of the sensitivity of fish, higher plants and other desirable aquatic lifeforms to most effective algicides.

We have now discovered that certain phosphine derivatives, known for use as bactericides and fungicides in industrial water treatment and plant protection, are highly effective at killing or inhibiting the growth of algae in the aforesaid ecologically sensitive water systems at concentrations at which they are non-toxic to fish, most higher aquatic plants and most similar aquatic flora and fauna. We have discovered, moreover, that treatment of water as aforesaid inhibits the spread of fungal, viral, bacterial or protozoal infections of fish and may aid the recovery of fish suffering from such infections.

According to one embodiment our invention provides a method for the treatment of ecologically sensitive water systems as herein defined which comprises adding thereto or maintaining therein an algicidal; non-ichthyotoxic concentration of a water-soluble phosphorus compound having the formula:

$$[HOR\ PR^1{}_n]_yX_x$$

where n is 2 or 3; x is 0 or 1, such that (n + x) = 2 or 4; y is 1 when x is zero or is equal to the valency of X when x is 1; R is an alkylene group of 1 to 4 carbon atoms; each $R^1$ is either HOR where R is as defined above, preferably a hydroxymethyl group, or else an alkyl or alkenyl group having from 1 to 4 carbon atoms; and X is a non-ichthyotoxic anion at the concentration used ; or a water-soluble condensate of any such phosphorus compound.

According to a second embodiment our invention provides a composition for the treatment of ecologically sensitive water systems as herein defined, which contains as active principal a phosphorus compound as aforesaid.

The phosphorus compounds suitable for use according to the present invention are those described in our GB 2 145 708 B and GB 2 182 563 A (the disclosures of which are incorporated herein by reference) and with the same preferences. In particular we prefer to use water-soluble tetrakis-(hydroxymethyl) phosphonium (hereinafter referred to as "THP") salts of non-ichthyotoxic anions, especially THP sulphate, THP chloride, THP phosphate and THP phosphite.

The compositions of our invention may typically contain from 0.01 to 95% by weight of the phosphorus compound, preferably from 0.1 to 90%, e.g. from 1 to 80%, more usually 10 to 75% and a solvent such as water or a carrier, diluent or controlled or sequential release agent such as a soluble glass or encapsulant, which will not harm fish when at the concentrations required.

The compositions may additionally or alternatively contain other non-ichthyotoxic biocides, synergists, surfactants, nutrients, corrosion inhibitors, flocculants and the like.

The compositions of our invention are typically dosed so as to maintain concentration of between 1 and 2000 ppm, preferably 2 to 1,000 ppm, more preferably 5 to 500 ppm, e.g. 10 to 100 ppm, more usually 12 to 50 ppm, most preferably 15 to 30 ppm of the phosphorus compound in the water.

The invention will be illustrated by the following example:

A fish pond contained 10 Koi Carp and water lilies. The water was circulated through a biological filter comprising a bed of gravel coated with bacteria to convert toxic nitrogenous products of fish metabolism to nitrate, and was recycled to the pond after oxygenation in a venturi.

One of the fish had exhibited signs of protozoal infection and the water was becoming infested with a voluminous growth of algae which was stifling the water lilies.

100 gms of a solution of 75% by weight THP sulphate in water was dosed slowly via the venturi to provide a concentration in the pond of 27 ppm of the solution.

After 48 hours the pool was highly turbid and 50% of the water was drained and the pool refilled with mains water. The same procedure for renewing the water was repeated at 72, 96 and 120 hours after dosing. After 10 days the algae had completely broken down and the water was clear.

Throughout this period the fish fed normally and showed no signs of distress. The infected fish recovered fully and no other fish became infected. The water lilies maintained normal growth and the biological filter continued to function satisfactorily.

## Claims

1. A method for the treatment of ecologically sensitive water-systems infested, or liable to infestation, with algae which comprises adding thereto or maintaining therein an algicidal, non-ichthyotoxic concentration of a water soluble phosphorus compound having the formula:

$$[HOPR^1_n]_y X_x$$

wherein n is 2 or 3; x is 0 or 1, such that (n + x) = 2 or 4; y is 1 when x is zero and is equal to the valency of X when x is 1; R is an alkylene group of 1 to 4 carbon atoms; each $R^1$ is either HOR where R is as defined above, or else an alkyl or alkenyl group having from 1 to 4 carbon atoms; and X is a non-ichthyotoxic anion at the concentration used; or a water-soluble condensate of any such phosphorus compound.

2. A method according to claim 1 wherein said phosphorus compound is a tetrakis (hydroxmethyl) phosphonium salt.

3. A method according to claim 1 wherein said phosphorus compound is a phosphate, sulphate, chloride or phosphite.

4. A method according to any foregoing claim wherein said concentration of said phosphorus compound is from 1 to 2000 ppm.

5. A method according to claim 4 wherein said concentration is between 10 and 100 ppm.

6. A method according to claim 5 wherein said concentration is from 12 to 50 ppm.

7. A method according to any foregoing claim wherein said water system contains, or is subsequently brought into contact with, live fish.

8. A method according to any foregoing claim wherein said water system is a fish pond, aquarium, fish farm, lake, river, canal, stream, water course or drainage or irrigation work.

9. A composition comprising an aqueous solution of a phosphorus compound as specified in claim 1 for use in the method of any foregoing claim.